# EUROPEAN PATENT SPECIFICATION

(11) **EP 3 436 103 B1**
(45) Date of publication and mention of the grant of the patent: **26.02.2020**
(21) Application number: 17724283.1
(22) Date of filing: 29.03.2017
(51) Int. Cl.: A61M 1/00, A61M 39/24

(54) **A DEVICE FOR THE PURIFICATION OF FAT EXTRACTED BY WAY OF SURGICAL LIPOSUCTION PROCEDURES**
VORRICHTUNG ZUR REINIGUNG VON DURCH CHIRURGISCHE FETTABSAUGUNG EXTRAHIERTES FETT
DISPOSITIF DE PURIFICATION DE GRAISSE EXTRAITE AU MOYEN DE PROCÉDURES CHIRURGICALES DE LIPOSUCCION

(30) Priority: 31.03.2016 IT UA20162142
(43) Date of publication of application: 06.02.2019
(73) Proprietor: Goisis, Mario, 20123 Milano (MI) (IT)
(72) Inventor: Goisis, Mario, 20123 Milano (MI) (IT)
(74) Representative: Pizzoli, Antonio
(86) International application number: PCT/IB2017/051789
(87) International publication number: WO 2017/168337

(56) References cited:
- WO-A1-2015/131087
- CN-A- 105 380 601
- US-A1- 2015 337 263

## Description

### Technical field of the invention

The present invention generally relates to regenerative medicine and surgery and in particular to a device for the purification of fat extracted by way of surgical liposuction procedures.

### Background

Liposuction has been introduced since the 70s of the last century. Initially it was a very invasive intervention carried out under general anesthesia in major surgery rooms. The procedure took several hours and fat in excess was sucked by way of large cannulas with a diameter ranging from 1 to 2 cm connected to a high-power suction unit. Fat was then eliminated along with large amounts of blood and anesthetic. In many cases, blood transfusions were required in the days following an intervention and many complications and deaths are reported in medical literature.

Less invasive liposuction techniques have been developed only since the 90s of the last century. The size of the cannulas has been progressively reduced up to diameters of 2-5 mm. The introduction of the tumescent anesthesia technique has allowed to carry out surgical interventions under local anesthesia. According to the tumescent technique a large volume of liquid containing a local anesthetic combined with a vasoconstrictor (adrenaline) is injected in an area to be treated. This injection reduces the pain caused to the patient during suction of fat and allows to limit bleeding, thus making liposuction easier and less dangerous.

The so-called "lipofilling" procedures have been developed over the past 20 years. These procedures exploit the sucked fat as a filler to reshape and increase of the volume of breast, gluteus and calf. Face wrinkles may also be filled and corrected, lips, cheekbones and chin may be reshaped as well.

Therefore it has become necessary to preserve the integrity of the sucked fat as much as possible. Unlike the first liposuction procedures, in fact, fat is no longer eliminated, but becomes a resource in the frame of the aesthetic procedure. The effectiveness of this resource is closely related to the survival of fat cells, i.e. adipocytes and stem cells and adipocyte precursor cells. These cells are taken from a site where they are in excess (thighs, abdomen, hips) and are grafted on a site to be reshaped (breasts, lips, etc.). The procedure is very delicate and is always accompanied by the death of a variable percentage of adipocytes. The survival rate ranges between 70%, in the better cases, and below 10%, which means failure of the lipofilling procedure.

The survival of fat cells is linked to several factors, such as their individual properties, the degree of oxidation of the tissues, the type of infiltration technique, the fact that the patient is a smoker, the trauma caused by a cannula during the sucking process, etc. The local anesthetic used to numb the surgical site is among the most important damaging factors for the adipocytes, because it has a toxic effect on the cells in the 12-24 hours subsequent to their graft into the target site. Damage is increased by the presence of erythrocytes that have an important oxidative effect if left with fat cells. In order to reduce tissue damage cannulas of a smaller size have recently been developed. These cannulas can be more easily inserted in the fatty tissue thus reducing the force to be applied. Cannulas having special patterns and shape of the holes through which fat is sucked have also been developed. An example of such cannulas is described in the Italian patent application no. 102015902323792 in the applicant's name.

During a liposuction procedure it is necessary to inject an anesthetic solution into the site from which fat has to be sucked. The anesthetic is usually injected by way of syringes.

Once made the anesthesia, suitable liposuction cannulas are mounted on the same syringes employed for this purpose and used to suck fat. The sucked fat is mixed with local anesthetic and blood. The difference in specific gravity is usually exploited as a means to separate adipocyte cells from the liquid mixture. To this aim the syringes are kept vertically manually or by way of suitable racks. Fat has a lower specific gravity and is collected in the upper part of a syringe, i.e. towards the plunger, while the liquid mixture is deposited in the lower part of the syringe, wherein an attachment member of the cannula is arranged, and can thus be eliminated.

In order to ensure survival of adipocyte cells in view of a subsequent lipofilling procedure, the fat remaining in the syringe must be processed with a washing solution, typically a physiological solution, for one or more cycles so as to purify it from residual blood and anesthetic. More particularly, during each cycle a certain amount of washing solution is sucked into the syringe, which is then positioned vertically so as to allow separation of fat and liquid mixture, the latter consisting of physiological solution, residual blood and anesthetic. The liquid mixture collected in the lower part of the syringe because of its higher specific gravity is then eliminated and fresh physiological solution is sucked into the syringe so as to carry out another washing and separation cycle.

A number of different containers are generally employed for the above operations, whose success is highly dependent on the manual ability of an operator. The containers must be properly prepared and organized before surgery. For example, a first container is filled with physiological or saline solution, a second container is filled with an anesthetic and a third container is used to collect the liquid mixture to be eliminated at each cycle of the purification process. These containers are generally open and thus exposed to a possible contamination by bacteria present in an operating room.

The Applicant has conceived a purification device for the fat extracted by way of surgical liposuction procedures. The device comprises a filter and a conduit having an attachment member allowing to receive a syringe containing fat extracted from a patient together with a liquid mixture formed of blood and anesthetic by way of a liposuction procedure. The conduit is arranged on one side of the filter. On the opposite side of the filter a first reservoir containing a physiological solution and a second, empty reservoir, are connected in parallel by a three-way attachment member. The first and the second reservoir may be in the form of syringes. The filter is configured to hold fat cells and let the liquid mixture pass. Such purification device is the object of the Italian pending patent application n. 102015000061679.

The device described above is configured such that by injecting the content of the syringe towards the filter the liquid mixture passing through it is directed and collected in the second, empty reservoir while, by actuating the plunger of the syringe in the opposite direction, the syringe sucks washing solution from the first reservoir through the filter. The washing solution is thus mixed with the fat and the residues contained in the syringe. A suitable system of one-way valves, taps and/or filters ensures tightness of the system, thus reducing the risk of contamination and subsequent infection.

By repeatedly cycling the injection and suction steps, the fat contained in the syringe is progressively purified from the residues of blood and anesthetic.

Although this fat purification device is extremely effective, the filter is progressively clogged, thus making it substantially impossible to suck washing solution from the first reservoir after a number of cycles and obliging a surgeon to replace the filter so as to complete the fat purification procedure.

In order to replace the filter it is necessary to interrupt the procedure and to disassemble and substitute a part of the device. Hence, the procedure has a higher cost and takes a longer time to be completed.

Also known are purification devices for fat extracted by way of liposuction procedures that comprise a manifold or conduit, a syringe connected or connectable in fluid communication with the manifold and intended to receive fat to be purified, a reservoir intended to contain a washing solution and a filter, wherein the reservoir is connected or connectable in fluid communication with the manifold through a one-way valve arranged such that a flow of fluid is possible towards the manifold. The syringe and the reservoir are connected or connectable to the manifold on a same side relative to the filter.

Thanks to this configuration it is possible to ensure supply of the washing solution even when the filter starts to be clogged, thus allowing a surgeon to complete a purification procedure without being obliged to interrupt it so as to replace the filter.

Examples of such devices are disclosed by US 2015/337263 A1, WO 2015/131087 A1 and CN 105380601 A.

### Summary of the invention

The invention is defined in the appended claims. The technical problem posed and solved by the present invention is to improve purification devices for fat extracted by liposuction in the aim of minimizing damages to fat cells when fat is subjected to a plurality of purification cycles e.g. by sucking it from a syringe and pumping it to a manifold and vice versa, as it happens in the devices disclosed by the patent publications US 2015/337263 A1, WO 2015/131087 A1 and CN 105380601 A mentioned above.

The purification device according to the invention comprises a manifold, i.e. a conduit or set of conduits, to which a first syringe containing fat to be purified and a second syringe, sack or more generally a reservoir containing a washing solution are connected in parallel. A filter is attached at one end of the manifold. The reservoir containing the washing solution is connected to the manifold through a one-way valve so that the washing solution can only flow towards the manifold, but not in the opposite direction, which would result in a contamination of the washing solution. A third syringe is connected to the manifold at the opposite end relative to the filter. Between the first manifold and the third syringe a bidirectional non-return valve is arranged. The bidirectional non-return valve has different pressure thresholds in the two flow directions, i.e. from the manifold to the third syringe and from the third syringe to the manifold. The configuration of the bidirectional non-return valve is such that the pressure threshold from the manifold to the third syringe is higher than the pressure threshold from the latter to the manifold.

Thanks to this configuration, it is possible to effectively prevent fat from flowing into the third syringe when the liquid mixture is eliminated and to have a threshold for the maximum pressure allowing to avoid damage to fat cells when the fat purified from the liquid mixture must be injected from the third syringe to the manifold so as to carry out one or more purification cycles.

According to an alternative embodiment of the invention, the non-return valve connecting the reservoir with the washing solution to the manifold is a dual non-return valve comprising a hollow body which encloses a movable closing element and three conduits branching off from the hollow body. The overall configuration of the dual non-return valve is such that a first fluid or substance can flow from a first conduit to a third conduit and in the opposite direction, while a second fluid or substance may flow from a second conduit to the third conduit but not in the opposite direction. A free end of the manifold is connected in fluid communication with the first conduit of the dual non-return valve, the reservoir is connected in fluid communication with the second conduit of the dual non-return valve and the third syringe is connected in fluid communication with the third conduit of the dual non-return valve.

The fat received in the first syringe is thus fed into the third syringe by crossing the dual non-return valve. The washing solution may be sucked by the third syringe from the reservoir through the dual non-return valve. Fat and washing solution are then pumped into the first syringe through the dual non-return valve and the liquid mixture is removed through the filter.

The dual non-return valve allows to limit the maximum pressure within the manifold, thus promoting survival of fat cells. More particularly, the dual non-return valve provides different pressure thresholds in the two opposite flow directions, thus effectively preventing fat from flowing into the third syringe when the liquid mixture is eliminated and allowing to have a threshold for the maximum pressure suitable to avoid damage to fat cells when fat is separated from the liquid mixture and must be subjected to one or more further purification cycles from the third syringe to the manifold.

It will be appreciated that operation of the device according to this alternative embodiment is the same as the embodiment described above and has the same advantages. In fact, the first conduit and the third conduit of the dual non-return valve form a bidirectional non-return valve.

The fat to be purified may advantageously be fed into the first syringe by way of a liposuction syringe that can be connected to the manifold at a suitable attachment member directly or through a conduit. The syringe is provided with a check valve such as a three-way tap, which allows the purification device to be isolated once the first syringe has been filled, while maintaining fluid communication within the manifold. This configuration provides the advantage of maintaining sterility of the elements of the purification device intended to carry out the fat purification process.

Still in the aim of maintaining the sterility of the elements intended to carry out the fat purification process, the purified fat may be fed from the third syringe to a lipofilling syringe or to a syringe for the infiltration of stem cells, which may be connected to the manifold at a suitable attachment member, directly or through a conduit, by way of a tap, e.g. a three-way tap.

According to a further aspect of the invention, the purification device may also advantageously comprise an assembly for mixing the purified fat with additives such as platelet-plated plasma (PRP), stromal cells, stem cells and/or precursors of adipocytes. It is well-known in fact that the combination of such additives and fat promotes grafting of the latter, because they are rich in growth factors. The mixing assembly comprises a second manifold to which a first syringe containing said additives is connected or connectable, and a second syringe intended to receive the purified fat from the device together with a measured amount of the additives.

Further advantages and features of the purification device according to the present invention will become clear to those skilled in the art from the following detailed and non-limiting description of embodiments thereof, with reference to the accompanying drawings.

### Brief description of the drawings

Reference will be made to the figures of the accompanying drawings, in which:
- Figure 1 schematically shows a fat purification device according to an embodiment of the invention;
- Figure 2 schematically shows a fat purification device according to an alternative embodiment of the invention;
- Figure 3 schematically shows a fat purification device according to a further embodiment of the invention.

### Detailed description of preferred embodiments of the invention

With reference to the figures, a purification device according to the invention is generally indicated by reference numeral 100.

The purification device 100 comprises a first manifold 110 extending in a longitudinal direction L and comprising a plurality of attachment members which stretch out in a transverse direction T, perpendicular to the longitudinal direction L. The attachment members are preferably of standard *"luer lock"* members configured to allow assembly of a plurality of syringes, sacks or reservoirs. In the figures, the attachment members are schematically shown by way of parallel lines and may advantageously include flow adjustment, check valves such as three-way taps as schematically shown in the figures.

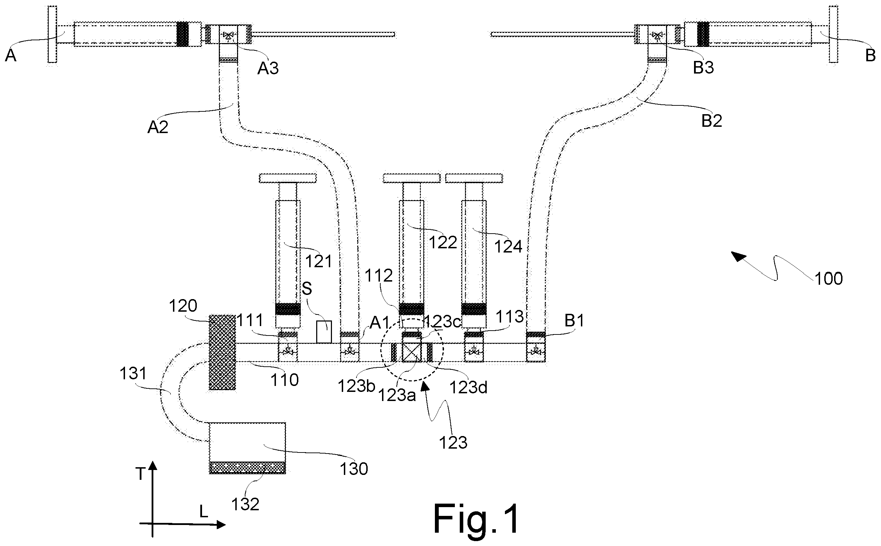

With particular reference to the embodiment shown in figure 1, the purification device 100 comprises a first syringe 121 intended to contain fat sucked from a patient during a liposuction procedure together with a liquid mixture consisting of local anesthetic and blood. The first syringe 121 is connected in fluid communication with the first manifold 110 at a first attachment member 111. The device 100 further comprises a reservoir 122, for example in the form of a second syringe, intended to contain a washing solution such as e.g. a physiological solution. The reservoir 122 is connected to the first manifold 110 at a second attachment 112 through a non-return valve 123 that allows a fluid to flow from the second syringe 122 to the manifold 110 but not in the opposite direction. Alternatively, the reservoir 122 may be connected to the first manifold 110 through a tap which, once closed manually, allows to prevent fluid to flow from the manifold 110 to the reservoir 122 in a totally equivalent way.

The device 100 further comprises at least one filter 120 connected to the first manifold 110 e.g. at one end thereof. The filter 120 is configured to hold fat cells and/or stem cells and precursors of adipose tissue and to allow the liquid mixture to pass. The filter 120 comprises meshes having apertures of a size comprised between 10 and 200 microns, preferably between 15 and 100 microns. The filter 120 may consist of several mesh filter elements of different size in order to optimize the purification process.

The device 100 also comprises a reservoir 130 connected to the filter 120 at the end opposite to the end restrained to the first manifold 110. The reservoir 130 is connected in fluid communication with the first manifold through the filter 120. In order to connect the reservoir 130 to the filter 120 a silicone conduit 131 may e.g. be employed, as schematically shown in figure 1 by way of a dotted line.

In operation the purification device 100 is arranged such that the first syringe 121 connected to the first manifold 110 is positioned substantially vertically, so that the fat to be purified, which has a lower specific gravity than the liquid mixture, is collected at the top of the syringe, i.e. at the plunger, while the liquid mixture is collected at the lower part of the syringe where this is attached to the manifold. The liquid mixture can thus be discharged. Consequently, in operation the first manifold 110 is arranged substantially horizontally.

In order to carry out a purification treatment of the fat collected in the first syringe 121 in view of a lipofilling treatment, by acting on the syringe plunger the whole fat content is injected into the first manifold 110 thus allowing to discharge the liquid mixture through the filter 120, which holds the fat cells. The liquid mixture is collected into the reservoir 130 connected to the filter 120. A certain amount of washing solution is then injected or sucked several times from the reservoir 122 and the fat mixed with the new liquid mixture is cyclically sucked into the first syringe 121 and pumped therefrom into the first manifold 110, thus discharging the liquid mixture through the filter 120 into the reservoir 130 until fat is completely purified. When fat is completely free from blood and anesthetic residues it has a straw yellow color. An element 132 made of or comprising an absorbent material may advantageously be arranged inside the reservoir 130, thus allowing an easier disposal of the liquid mixture.

It will be appreciated that, unlike the configuration of the purification device described in the co-pending application 102015000061679, in the purification device 100 according to the invention the syringe 121 containing fat and the reservoir 122 containing the washing solution are arranged on a same side of the first manifold 110 relative to the filter 120, thus allowing to complete a purification procedure even when the filter becomes clogged.

As shown in the embodiment of the invention of Figure 1, the first syringe 121 is initially empty and receives the fat to be purified from a liposuction syringe A which can be connected to the first manifold 110 at a further attachment member A1 thereof through a conduit A2 and an a check valve A3 such as e.g. a three-way tap which, once the first syringe 121 has been filled, interrupts the fluid communication between the liposuction syringe A and the first manifold 110, while ensuring the continuity of the flow inside the manifold.

This configuration is advantageous in that no external elements need to be assembled on the purification device 100. Hence, sterility of all the components intended to be involved in the fat purification process is ensured.

According to this embodiment, when fat is fed from the liposuction syringe A to the first manifold 110, the liquid mixture is eliminated through the filter 120 while fat gradually fills the first syringe 121, thereby reducing the overall time of the purification cycle.

In the embodiment of the invention shown in figure 1, the non-return valve 123 is in particular a dual non-return valve comprising a hollow body 123a which encloses a movable locking element (not shown), and three conduits 123b, 123c, 123d branching off from said hollow body 123a. The overall configuration of the dual non-return valve 123 is such that a first fluid or substance, such as a mixture of liquid and fat, can flow from a first conduit 123b to a third conduit 123d and in the opposite direction, while a second fluid or substance, such as a washing solution, can flow from a second conduit 123c to the third conduit 123d, but not in the opposite direction.

Dual non-return of the above type are known in the field of catheters for surgical instruments and related accessories and are commercially available.

The purification device 100 further comprises a third syringe 124 and the overall configuration is such that a free end of the first manifold 110, opposite to the filter 120 in the illustrated embodiment, is connected in fluid communication with the first conduit 123b of the dual non-return valve 123, the reservoir 122 is connected in fluid communication with the second conduit 123c of the dual non-return valve 123 and the third syringe 124 is connected in fluid communication with the third conduit 123d of the dual non-return valve 123.

The third syringe 124 may be connected to the dual non-return valve 123 directly, or, as shown in figure 1, through an tail portion of the first manifold 110 and possibly also a by way of a shut-off valve, such as a three-way tap.

According to this embodiment of the invention, fat and washing solution are fed into the third syringe 124 through the first conduit 123b of the dual non-return valve 123 and injected together through the third conduit 123d into the first manifold 110, where fat is received into the first syringe 121 and is separated from the liquid mixture, which is discharged through the filter 120.

This configuration of the device is advantageous because the dual non-return valve 123 allows to limit the maximum pressure inside the first manifold 110 thus promoting the survival of fat cells and hence contributing to the success of a subsequent lipofilling treatment.

This configuration is advantageous also because dual non-return valves typically have different opening pressure thresholds in the two flow directions, in this case from the first manifold 110 to the third syringe 124 and from the latter to the first manifold 110. The first conduit 123b and the third conduit 123d are configured such that the pressure threshold from the first manifold 110 to the third syringe 124, i.e. from the first conduit 123b to the third conduit 123d, is higher than the pressure threshold from the latter towards the first manifold 110, i.e. from the third conduit 123d to the first conduit 123b, thereby effectively preventing the flow of fat to the third syringe 124 when the liquid mixture is separated from the fat and to have a threshold for the maximum pressure so as to avoid to damage fat cells when the fat free from the liquid mixture is subjected to one or more further purification cycles from the third syringe 124 to the first manifold 110.

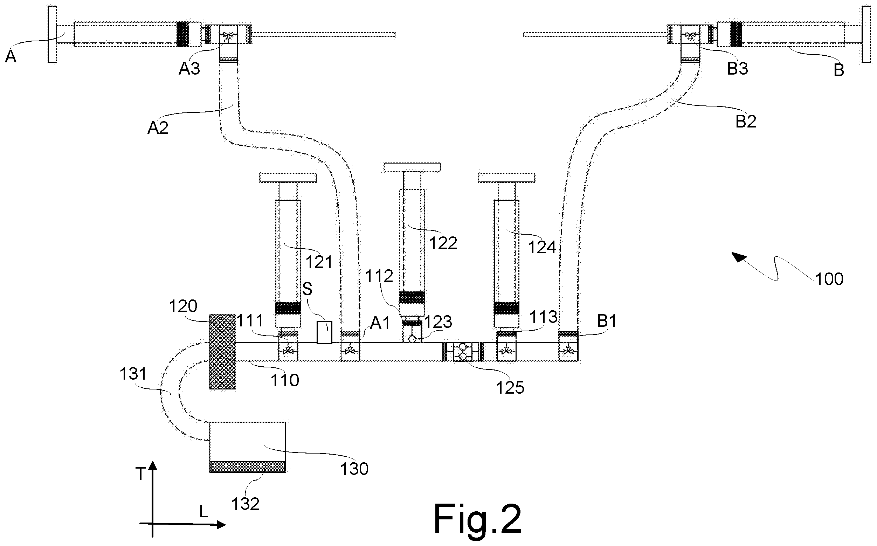

It will be appreciated that the choice of a dual non-return valve is not a limiting feature of the invention. As schematically shown in the alternative embodiment of figure 2, the reservoir 122 may be connected to the first manifold 110 by way of a conventional one-way valve 123 or a tap, and a bidirectional non-return valve 125, commercially known in the field of medical devices, may be arranged between the first manifold 110 and the third syringe 124.

The bidirectional non-return valve 125 has different opening pressure thresholds in the two flow directions, i.e. from the first manifold 110 to the third syringe 124 and from the latter to the first manifold 110. Similarly to the embodiment of figure 1, the arrangement of the bidirectional non-return valve 125 is such that the pressure threshold from the first manifold 110 to the third syringe 124 is higher than the pressure threshold from the latter towards the first manifold 110, thus effectively preventing flow of fat toward the third syringe 124 when the liquid mixture is separated and allowing to have a threshold for the maximum pressure so as to avoid to damage to fat cells when fat is separated from the liquid mixture and must be subjected to one or more further purification cycles from the third syringe 124 to the first manifold 110.

In other words, the bidirectional non-return valve 125 performs the same function as the first conduit 123b and the third conduit 123d of the dual non-return valve 123 described above, or conversely, the first conduit 123b and the third conduit 123d of the dual non-return 123 valve form or constitute a bidirectional non-return valve.

The fat so purified may be advantageously fed to a lipofilling syringe B, which can be connected to the first manifold 110 at a further attachment member B1, e.g. arranged at its free end. The lipofilling syringe B can be connected to the attachment member B1, directly of by way of an additional conduit B2, through a tap B3, such as a three-way tap. This configuration is advantageous in that it does not require to disassemble the purification device 100, thereby ensuring sterility of all the components involved in the fat purification process. As described above in connection with the attachment of the liposuction syringe A, the attachment member B1 may advantageously be provided with a check valve B3, for example a three-way tap, which allows to interrupt the fluid communication between the lipofilling syringe B and the first manifold 110 during the operation steps described above.

The first manifold 110 of the device 100 according to the invention can advantageously be placed on a vibrating platform (not shown) which promotes separation between the various components of fat (adipocytes, stem and stromal cells, liquid) during the purification process carried out according to the operation mode and the device configurations disclosed above.

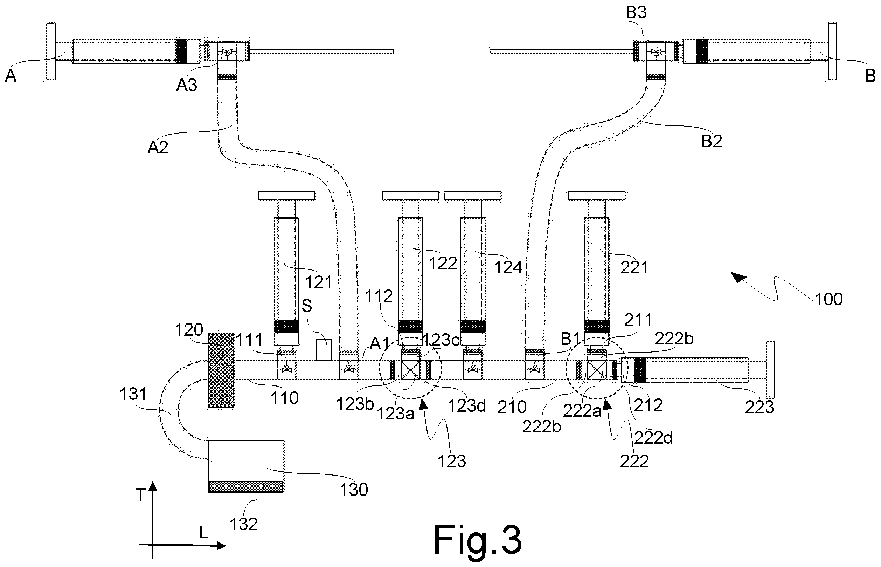

Now referring to figure 3, according to a further embodiment of the invention the purification device 100 may advantageously comprise a mixing assembly for the mixing of purified fat with additives, such as in particular platelet-rich plasma, stromal cells, stem cells and/or precursors of adipocytes. It is well known that the combination of such additives that are rich in growth factors and fat promotes grafting of fat in lipofilling treatments.

The mixing assembly for the mixing of purified fat with additives includes a second manifold 210 which can be connected to a free end of the first manifold 110, for example the end opposite to the filter 120. The mixing group further comprises a reservoir 221, for example in the form of a first syringe intended to contain said additives and connected or connectable in fluid communication with the second manifold 210 at a first attachment member 211 thereof. The connection is made through a non-return valve 222 arranged so as to allow a fluid to flow from the reservoir 221 to the second manifold 210, but not in the opposite direction. The mixing assembly further comprises a second syringe 223 connected or connectable in fluid communication with the second manifold 210 at a second attachment member 212 thereof and intended to receive purified fat from the purification device 100 and additives from the reservoir 221.

According to a preferred embodiment of the invention, the non-return valve 222 is a dual non-return valve as described above, The valve comprises a hollow body 222a enclosing a movable closing element (not shown), and three conduits 222b, 222c, 222d which branch off from the hollow body 222a. The overall configuration of the mixing assembly is such that the end of the second manifold 210 opposite to the end connected to the first manifold 110 is set in fluid communication with the first conduit 222b of the dual non-return valve 222, the reservoir 221 is set in fluid communication with the second conduit 222c of the dual non-return valve 222 and the second syringe 223 is set in fluid communication with the third conduit 222d of the dual non-return valve 222.

The use of a dual non-return valve 222 is advantageous because, as explained above, it is possible to limit the pressure inside the manifold thus promoting survival of the fat cells enriched with the additives and raising the possibility of success of subsequent lipofilling procedures.

It will be appreciated that also in this case alternatively to the use of a non-return valve a conventional check valve coupled to a bidirectional non-return valve arranged between the reservoir 221 and the second syringe 223 may be used.

According to a further aspect of the invention, the purification device 100 can advantageously comprise a sensor S configured to measure the relative percentages of fat and washing solution. The sensor S is mounted in a branch configuration on the first manifold 110 and may e.g. include an electrical impedance, an infrared rays measuring device of the type NIR (Near-Infrared Interactance), an electromagnetic field generator of the type TOBEC (Total Body Electrical Conductivity), or an X-ray system of the type DEXA (Dual Energy X-Ray Absorptiometry).

The invention has herein been disclosed with reference to preferred embodiments thereof. It will be appreciated that further embodiments based on the same inventive idea may exist as defined by the scope of protection of the claims set out below.

## Claims

1. A device (100) for the purification of fat extracted by way of liposuction procedures, said device (100) comprising:
i) a first manifold (110);
ii) a first syringe (121) intended to receive fat to be purified, said first syringe (121) being connected or connectable in fluid communication with said first manifold (110) at a first attachment member (111) thereof;
iii) a reservoir (122) intended to contain a washing solution, said reservoir (122) being connected or connectable in fluid communication with the first manifold (110) at a second attachment member (112) thereof through a non-return valve (123), said non-return valve (123) being arranged so as to allow a fluid to flow from the reservoir (122) towards the first manifold (110);
iv) at least one filter (120) connected to an end portion of the first manifold (110),
wherein said first syringe (121) and said reservoir (122) are connected or connectable to the first manifold (110) on a same side thereof with respect to said filter (120), **characterised in that** the device comprises a third syringe connected at the opposite end portion of the first manifold (110),
and wherein a non-return bidirectional valve (125) is arranged between the first manifold (110) and said third syringe (124), said non-return bidirectional valve (125) having different opening pressure thresholds in the two opposite flow directions and being arranged such that the pressure threshold from the first manifold (110) to the third syringe (124) is higher than the pressure threshold from the latter to the first manifold (110).

2. A purification device (100) according to claim 1, wherein said non-return valve (123) is a dual non-return valve comprising a hollow body (123a), which encloses a movable closure element and three conduits (123b, 123c, 123d) that branch off from said hollow body (123a), the overall configuration of the dual non-return valve (123) being such that a first fluid or substance can flow from a first conduit (123b) to a third conduit (123d) and in the opposite direction, while a second fluid or substance can flow from a second conduit (123c) to the third conduit (123d), but not in the opposite direction, wherein a free end of the first manifold (110) is connected in fluid communication with the first conduit (123b) of the dual non-return valve (123), the reservoir (122) is connected in fluid communication with the second conduit (123c) of the dual non-return valve (123) and the third syringe (124) is connected in fluid communication with the third conduit (123d) of the dual non-return valve (123), and wherein the first conduit (123b) and the third conduit (123d) are configured such that a pressure threshold from the first manifold (110) to the third syringe (124) is higher than a pressure threshold from the latter to the first manifold (110), the first conduit (123b) and the third conduit (123d) of the dual non-return valve (123) forming the non-return bidirectional valve (125).

3. A purification device (100) according to claim 1 or 2, wherein said filter (120) comprises meshes with an apertures size comprised between 10 and 200 microns, preferably between 15 and 100 microns.

4. A purification device (100) according to any one of claims 1 to 3, further comprising a reservoir (130) connected or connectable to the filter (120) at the end thereof opposite to the end connected to the first manifold (110), said reservoir (130) being in fluid communication with the latter through the filter (120).

5. A purification device (100) according to any one of claims 1 to 4, further comprising a liposuction syringe (A) connectable to the first manifold (110) in correspondence of a further attachment member (A1) thereof.

6. A purification device (100) according to any one of claims 1 to 5, further comprising a lipofilling syringe (B) connectable to the first manifold (110) in correspondence with still a further attachment member (B1) thereof.

7. A purification device (100) according to any one of claims 1 to 6, further comprising a vibrating platform, the first manifold (110) being mounted on said vibrating platform.

8. A purification device (100) according to any one of claims 1 to 7, further comprising a mixing assembly for the mixing of purified fat with additives, said mixing assembly comprising a second manifold (210) connectable to the first manifold (110) at a free end thereof, a reservoir (221) intended to contain said additives being connected or connectable in fluid communication with said second manifold (210) in correspondence of a first attachment member (211) thereof through a non-return valve (222), said non-return valve (222) being arranged so as to allow a flow of fluid from said reservoir (221) towards the second manifold (210), the mixing assembly further comprising a second syringe (223), intended to receive purified fat and additives, being connected or connectable in fluid communication with the second manifold (210) in correspondence with a second attachment member (212) thereof.

9. A purification device (100) according to claim 8, wherein said non-return valve (222) is a dual non-return valve comprising a hollow body (222a), which encloses a movable closure element, and three conduits (222b, 222c, 222d) that branch off from said hollow body (222a), the overall configuration of the dual non-return valve (222) being such that a first fluid or substance can flow from a first conduit (222b) to a third conduit (222d) and in the opposite direction, while a second fluid or substance can flow from a second conduit (222c) to the third conduit (222d), but not in the opposite direction, wherein the end the second manifold (210) opposite to the end connected to the first manifold (110) is connected in fluid communication with the first conduit (222b) of the dual non-return valve (222), the reservoir (221) is connected in fluid communication with the second conduit (222c) of the dual non-return valve (222) and the second syringe (223) is connected in fluid communication with the third conduit (222d) of the dual non-return valve (222).

## Patentansprüche

1. Eine Vorrichtung (100) zur Reinigung von durch Fettabsaugung extrahiertem Fett, wobei die Vorrichtung (100) umfasst:
i) ein erstes Sammelrohr (110);
ii) eine erste Spritze (121), dazu bestimmt, zu reinigendes Fett zu empfangen, wobei die besagte erste Spritze (121) an dem besagten ersten Sammelrohr (110) über ein erstes Befestigungsstück desselben in einer Strömungsverbindung angeschlossen oder anschließbar ist;
iii) ein Reservoir (122), dazu bestimmt, eine Waschlösung aufzunehmen, wobei das besagte Reservoir (122) an dem besagten ersten Sammelrohr (110) an einem zweiten Befestigungsstück desselben über ein Rückschlagventil (123) in einer Strömungsverbindung angeschlossen oder anschließbar ist, wobei das besagte Rückschlagventil (123) derart angeordnet ist, dass es einem Fluid ermöglicht, von dem Reservoir (122) zu dem ersten Sammelrohr (110) zu fließen;
iv) wenigstens einen Filter (120), der an einem Endbereich des ersten Sammelrohrs (110) angeschlossen ist,
wobei die besagte erste Spritze (121) und das besagte Reservoir (122) an dem ersten Sammelrohr (110) an der gleichen Seite desselben in Bezug auf den besagten Filter (120) angeschlossen oder anschließbar sind, **dadurch gekennzeichnet, dass** die Vorrichtung eine dritte Spritze aufweist, die an dem gegenüber liegenden Endbereich des Sammelrohrs (110) angeschlossen ist,
und wobei ein bidirektionales Rückschlagventil (125) zwischen dem ersten Sammelrohr (110) und der besagten dritten Spritze (124) angeordnet ist, wobei das besagte bidirektionale Rückschlagventil (125) unterschiedliche Öffnungsdruckschwellen in den beiden entgegengesetzten Strömungsrichtungen aufweist und derart angeordnet ist, dass die Druckschwelle von dem ersten Sammelrohr (110) zu der dritten Spritze (124) höher ist als die Druckschwelle von der letzteren zu dem ersten Sammelrohr (110).

2. Eine Reinigungsvorrichtung (100) gemäß Anspruch 1, wobei das besagte Rückschlagventil (123) ein duales Rückschlagventil ist, umfassend einen hohlen Körper (123a), der ein bewegliches Verschlusselement aufnimmt sowie drei Leitungen (123b, 123c, 123d), welche von dem hohlen Körper (123a) abzweigen, wobei die Gesamtkonfiguration des dualen Rückschlagventils (123) derart ist, dass ein erstes Fluid oder eine erste Substanz von einer ersten Leitung (123b) zu einer dritten Leitung (123d) strömen kann sowie in der entgegengesetzten Richtung, während ein zweites Fluid oder eine zweite Substanz von einer zweiten Leitung (123c) zu der dritten Leitung (123d) strömen kann, aber nicht in der entgegengesetzten Richtung, wobei ein freies Ende des ersten Sammelrohrs (110) in einer Strömungsverbindung mit der ersten Leitung (123b) des dualen Rückschlagventils (123) verbunden ist, wobei das Reservoir (122) in einer Strömungsverbindung an der zweiten Leitung (123c) des dualen Rückschlagventils (123) angeschlossen ist, und wobei die dritte Spritze (124) in einer Strömungsverbindung an der dritten Leitung (123d) des dualen Rückschlagventils (123) angeschlossen ist, und wobei die erste Leitung (123b) und die dritte Leitung (123d) derart konfiguriert sind, dass eine Druckschwelle von der ersten Sammelleitung (110) zu der dritten Spritze (124) höher ist als eine Druckschwelle von der letzteren zu der ersten Sammelleitung (110), wobei die erste Leitung (123b) und die dritte Leitung (123d) des dualen Rückschlagventils (123) das bidirektionale Rückschlagventil (125) bilden.

3. Eine Reinigungsvorrichtung (100) gemäß Anspruch 1 oder 2, wobei der besagte Filter (120) Maschen mit einer Öffnungsweite zwischen 10 und 200 Mikron aufweist, vorzugsweise zwischen 15 und 100 Mikron.

4. Eine Reinigungsvorrichtung (100) gemäß einem der Ansprüche 1 bis 3, ferner umfassend ein Reservoir (130), das an dem Filter (120) angeschlossen oder anschließbar ist an dessen dem mit dem ersten Sammelrohr (110) verbundenen Ende gegenüber liegenden Ende, wobei das Reservoir (130) mit dem letzteren über den Filter (120) in Strömungsverbindung steht.

5. Eine Reinigungsvorrichtung (100) gemäß einem der Ansprüche 1 bis 4, ferner umfassend eine Fettabsaugungsspritze (A), die an dem ersten Sammelrohr (110) in Kontakt mit einem weiteren Befestigungsstück (A1) desselben anschließbar ist.

6. Eine Reinigungsvorrichtung (100) gemäß einem der Ansprüche 1 bis 5, ferner umfassend eine Fetttransplantationsspritze (B), die an dem ersten Sammelrohr (110) in Kontakt mit einem noch weiteren Befestigungsstück (B1) desselben anschließbar ist.

7. Eine Reinigungsvorrichtung (100) gemäß einem der Ansprüche 1 bis 6, ferner umfassend eine Rüttelplattform, wobei das erste Sammelrohr (110) auf der besagten Rüttelplattform montiert ist.

8. Eine Reinigungsvorrichtung (100) gemäß einem der Ansprüche 1 bis 7, ferner umfassend eine Mischeinrichtung zum Mischen von gereinigtem Fett mit Additiven, wobei die besagte Mischeinrichtung ein zweites Sammelrohr (210) umfasst, das mit dem ersten Sammelrohr (110) an einem freien Ende desselben verbinbar ist, ein Reservoir (221), dazu bestimmt, die besagten Additive aufzunehmen, welches an dem besagten zweiten Sammelrohr (210) in Kontakt mit einem ersten Befestigungsstück (211) desselben über ein Rückschlagventil (222) in einer Strömungsverbindung angeschlossen oder anschließbar ist, wobei das besagte Rückschlagventil (222) derart angeordnet ist, dass es eine Strömung eines Fluids von dem besagten Reservoir (221) in Richtung zu dem zweiten Sammelrohr (210) zulässt, wobei die Mischeinrichtung ferner eine zweite Spritze (223) umfasst, dazu bestimmt, gereinigtes Fett und Additive aufzunehmen, welche an dem zweiten Sammelrohr (210) in Kontakt mit einem zweiten Befestigungsstück (212) desselben in einer Strömungsverbindung angeschlossen oder anschließbar ist.

9. Eine Reinigungsvorrichtung (100) gemäß Anspruch 1, wobei das besagte Rückschlagventil (222) ein duales Rückschlagventil ist, umfassend einen hohlen Körper (222a), der ein bewegliches Verschlusselement aufnimmt, sowie drei Leitungen (222b, 222c, 222d), welche von dem hohlen Körper (222a) abzweigen, wobei die Gesamtkonfiguration des dualen Rückschlagventils (222) derart ist, dass ein erstes Fluid oder eine erste Substanz von einer ersten Leitung (222b) zu einer dritten Leitung (222d) strömen kann, sowie in der entgegengesetzten Richtung, während ein zweites Fluid oder eine zweite Substanz von einer zweiten Leitung (222c) zu der dritten Leitung (222d) strömen kann, aber nicht in der entgegengesetzten Richtung, wobei das dem mit dem ersten Sammelrohr (110) verbundenen Ende gegenüber liegende Ende des zweiten Sammelrohrs (210) an der ersten Leitung (222b) des dualen Rückschlagventils (222) in einer Strömungsverbindung angeschlossen ist, wobei das Reservoir (221) in einer Strömungsverbindung an der zweiten Leitung (222c) des dualen Rückschlagventils (222) angeschlossen ist, und wobei die zweite Spritze (223) an der dritten Leitung (222d) des dualen Rückschlagventils (222) in einer Strömungsverbindung angeschlossen ist.

## Revendications

1. Dispositif (100) pour la purification de graisse extraite au moyen de procédures de liposuccion, ledit dispositif (100) comprenant :
i) un premier collecteur (110) ;
ii) une première seringue (121) prévue pour recevoir la graisse à purifier, ladite première seringue (121) étant raccordée ou pouvant être raccordée en communication de fluide avec ledit premier collecteur (110) au niveau de son premier élément de fixation (111),
iii) un réservoir (122) prévu pour contenir une solution de lavage, ledit réservoir (122) étant raccordé ou pouvant être raccordé en communication de fluide avec le premier collecteur (110) au niveau de son second élément de fixation (112) par une valve de non-retour (123), ladite valve de non-retour (123) étant agencée afin de permettre l'écoulement d'un fluide du réservoir (122) vers le premier collecteur (110) ;
iv) au moins un filtre (120) raccordé à une partie d'extrémité du premier collecteur (110),
dans lequel ladite première seringue (121) et ledit réservoir (122) sont raccordés ou peuvent être raccordés au premier collecteur (110) du même côté de ce dernier par rapport audit filtre (120), **caractérisé en ce que** le dispositif comprend une troisième seringue raccordée au niveau de la partie d'extrémité opposée du premier collecteur (110),
et dans lequel une valve bidirectionnelle de non-retour (125) est agencée entre le premier collecteur (110) et ladite troisième seringue (124), ladite valve bidirectionnelle de non-retour (125) ayant des seuils de pression d'ouverture différents dans les deux directions d'écoulement opposées et étant agencée de sorte que le seuil de pression, du premier collecteur (110) à la troisième seringue (124), est supérieur au seuil de pression de cette dernière au premier collecteur (110).

2. Dispositif de purification (100) selon la revendication 1, dans lequel ladite valve de non-retour (123) est une double valve de non-retour comprenant un corps creux (123a) qui enferme un élément de fermeture mobile et trois conduits (123b, 123c, 123d) qui bifurquent dudit corps creux (123a), la configuration globale de la double valve de non-retour (123) étant telle qu'un premier fluide ou substance peut s'écouler d'un premier conduit (123b) à un troisième conduit (123d) et dans la direction opposée, alors qu'un second fluide ou substance peut s'écouler d'un deuxième conduit (123c) au troisième conduit (123d), mais pas la direction opposée, dans lequel une extrémité libre du premier collecteur (110) est raccordée en communication de fluide avec le premier conduit (123b) de la double-valve de non-retour (123), le réservoir (122) est raccordé en communication de fluide avec le deuxième conduit (123c) de la double-valve de non-retour (123) et la troisième seringue (124) est raccordée en communication de fluide avec le troisième conduit (123d) de la double-valve de non-retour (123), et dans lequel le premier conduit (123b) et le troisième conduit (123d) sont configurés de sorte qu'un seuil de pression, du premier collecteur (110) à la troisième seringue (124), est supérieur à un seuil de pression de cette dernière au premier collecteur (110), le premier conduit (123b) et le troisième conduit (123d) de la double-valve de non-retour (123) formant la valve bidirectionnelle de non-retour (125).

3. Dispositif de purification (100) selon la revendication 1 ou 2, dans lequel ledit filtre (120) comprend des mailles avec une taille d'ouverture comprise entre 10 et 200 microns, de préférence entre 15 et 100 microns.

4. Dispositif de purification (100) selon l'une quelconque des revendications 1 à 3, comprenant en outre un réservoir (130) raccordé ou pouvant être raccordé au filtre (120) au niveau de son extrémité opposée à l'extrémité raccordée au premier collecteur (110), ledit réservoir (130) étant en communication de fluide avec ce dernier par le biais du filtre (120).

5. Dispositif de purification (100) selon l'une quelconque des revendications 1 à 4, comprenant en outre une seringue de liposuccion (A) pouvant être raccordée au premier collecteur (110) en correspondance avec son autre élément de fixation (A1).

6. Dispositif de purification (100) selon l'une quelconque des revendications 1 à 5, comprenant en outre une seringue de liporemplissage (B) pouvant être raccordée au premier collecteur (110) en correspondance avec encore son autre élément de fixation (B1).

7. Dispositif de purification (100) selon l'une quelconque des revendications 1 à 6, comprenant en outre une plateforme vibrante, un premier collecteur (110) étant monté sur ladite plateforme vibrante.

8. Dispositif de purification (100) selon l'une quelconque des revendications 1 à 7, comprenant en outre un ensemble de mélange pour le mélange de la graisse purifiée avec des additifs, ledit ensemble de mélange comprenant un second collecteur (210) pouvant être raccordé au premier collecteur (110) au niveau de son extrémité libre, un réservoir (221) prévu pour contenir lesdits additifs étant raccordé ou pouvant être raccordé, en communication de fluide, avec ledit second collecteur (210) en correspondance de son premier élément de fixation (211) par le biais d'une valve de non-retour (222), ladite valve de non-retour (222) étant agencée pour permettre un écoulement de fluide dudit réservoir (221) vers le second collecteur (210), l'ensemble de mélange comprenant en outre une deuxième seringue (223) prévue pour recevoir la graisse purifiée et les additifs, qui est raccordée ou qui peut être raccordée en communication de fluide avec le second collecteur (210) en correspondance avec son second élément de fixation (212).

9. Dispositif de purification (100) selon la revendication 8, dans lequel ladite valve de non-retour (222) est une double-valve de non-retour comprenant un corps creux (222a) qui enferme un élément de fermeture mobile, et trois conduits (222b, 222c, 222d) qui bifurquent dudit corps creux (222a), la configuration globale de la double-valve de non-retour (222) étant telle qu'un premier fluide ou substance peut s'écouler à partir d'un premier conduit (222b) jusqu'à un troisième conduit (222d) et dans la direction opposée, alors qu'un second fluide ou substance peut s'écouler d'un deuxième conduit (222c) au troisième conduit (222d) mais pas la direction opposée, dans lequel l'extrémité du second collecteur (210) opposée à l'extrémité raccordée au premier collecteur (110) est raccordée, en communication de fluide, avec le premier conduit (222b) de la double-valve de non-retour (222), le réservoir (221) est raccordé en communication de fluide avec le deuxième conduit (222c) de la double-valve de non-retour (222) et la deuxième seringue (223) est raccordée en communication de fluide avec le troisième conduit (222d) de la double-valve de non-retour (222).
